# EUROPEAN PATENT SPECIFICATION

(11) **EP 1 200 473 B1**
(45) Date of publication and mention of the grant of the patent: **10.10.2007**
(21) Application number: 00952094.1
(22) Date of filing: 20.07.2000
(51) Int. Cl.: C07K 14/705, G01N 33/566, C12N 5/10, C12N 15/09

(54) **G-Protein coupled RECEPTOR**
G-Protein gekoppelter REZEPTOR
RECEPTEUR

(30) Priority: 21.07.1999 SE 9902763
(43) Date of publication of application: 02.05.2002
(73) Proprietor: AstraZeneca AB, 151 85 Södertälje (SE)
(72) Inventor: AHMAD, Sultan, St. Laurent, Quebec, Montreal H4S 1Z9 (CA); HOFFERT, Cyrla, St. Laurent, Quebec, Montreal H4S 1Z9 (CA); O'DONNELL, Dajan, St. Laurent, Quebec, Montreal H4S 1Z9 (CA); PELLETIER, Manon, St. Laurent, Quebec, Montreal H4S 1Z9 (CA); WALKER, Philippe, St. Laurent, Quebec, Montreal H4S 1Z9 (CA)
(86) International application number: PCT/SE2000/001505
(87) International publication number: WO 2001/005829

(56) References cited:
- WO-A1-00/11166
- WO-A2-99/19513
- DATABASE EMROD ACC. NO. AF233649, 24 February 2000 IM D.-S. ET AL.: 'Characterization of a novel sphingosine 1-phosphate receptor, Edg-8', XP002934508 & J. BIOL. CHEM. vol. 275, no. 19, 2000, pages 14281 - 14286
- MICHELLE GLICKMAN ET AL.: 'Molecular cloning, tissue-specific expression and chromosomal localization of a novel nerve growth factor-regulated G-protein-coupled receptor, nrg-1' MOLECULAR AND CELLULAR NEUROSCIENCE vol. 14, August 1999, pages 141 - 152, XP002934509

### SEQUENCE LISTING

<110> Astra Pharma Inc.
   Ahmad, Sultan
   Hoffert, Cyrla
   O'Donnell, Dajan
   Pelletier, Manon
   Walker, Philippe
<120> B1C3 G Protein-Coupled Receptor
<130> walker 6
<140>
   <141>
<160> 6
<170> PatentIn Ver. 2.0
<210> 1
   <211> 400
   <212> PRT
   <213> Rat
<400> 1
   Met Glu Ser Gly Leu Leu Arg Pro Ala Pro Val Ser Glu Val Ile Val
    1               5                  10                  15
   Leu His Tyr Asn Tyr Thr Gly Lys Leu Arg Gly Ala Arg Tyr Gln Pro
               20                  25                  30
   Gly Ala Gly Leu Arg Ala Asp Ala Ala Val Cys Leu Ala Val Cys Ala
           35                  40                  45
   Phe Ile Val Leu Glu Asn Leu Ala Val Leu Leu Val Leu Gly Arg His
       50                  55                  60
   Pro Arg Phe His Ala Pro Met Phe Leu Leu Leu Gly Ser Leu Thr Leu
   65                  70                  75                  80
   Ser Asp Leu Leu Ala Gly Ala Ala Tyr Ala Thr Asn Ile Leu Leu Ser
                   85                  90                  95
   Gly Pro Leu Thr Leu Arg Leu Ser Pro Ala Leu Trp Phe Ala Arg Glu
               100                 105                 110
   Gly Gly Val Phe Val Ala Leu Ala Ala Ser Val Leu Ser Leu Leu Ala
           115                 120                 125
   Ile Ala Leu Glu Arg His Leu Thr Met Ala Arg Arg Gly Pro Ala Pro
       130                 135                 140
   Ala Ala Ser Arg Ala Arg Thr Leu Ala Met Ala Val Ala Ala Trp Gly
   145                 150                 155                 160
   Leu Ser Leu Leu Leu Gly Leu Leu Pro Ala Leu Gly Trp Asn Cys Leu
                   165                 170                 175
   Gly Arg Leu Glu Ala Cys Ser Thr Val Leu Pro Leu Tyr Ala Lys Ala
               180                 185                 190
   Tyr Val Leu Phe Cys Val Leu Ala Phe Leu Gly Ile Leu Ala Ala Ile
           195                 200                 205
   Cys Ala Leu Tyr Ala Arg Ile Tyr Cys Gln Val Arg Ala Asn Ala Arg
       210                 215                 220
   Arg Leu Arg Ala Gly Pro Gly Ser Arg Arg Ala Thr Ser Ser Ser Arg
   225                 230                 235                 240
   Ser Arg His Thr Pro Arg Ser Leu Ala Leu Leu Arg Thr Leu Ser Val
                   245                 250                 255
   Val Leu Leu Ala Phe Val Ala Cys Trp Gly Pro Leu Phe Leu Leu Leu
               260                 265                 270
   Leu Leu Asp Val Ala Cys Pro Ala Arg Ala Cys Pro Val Leu Leu Gln
           275                 280                 285
   Ala Asp Pro Phe Leu Gly Leu Ala Met Ala Asn Ser Leu Leu Asn Pro
       290                 295                 300
   Ile Ile Tyr Thr Phe Thr Asn Arg Asp Leu Arg His Ala Leu Leu Arg
   305                 310                 315                 320
   Leu Leu Cys Cys Gly Arg Gly Pro Cys Asn Gln Asp Ser Ser Asn Ser
                   325                 330                 335
   Leu Gln Arg Ser Pro Ser Ala Val Gly Pro Ser Gly Gly Gly Leu Arg
               340                 345                 350
   Arg Cys Leu Pro Pro Thr Leu Asp Arg Ser Ser Ser Pro Ser Glu His
           355                 360                 365
   Ser Cys Pro Gln Arg Asp Gly Met Asp Thr Ser Cys Ser Thr Gly Ser
       370                 375                 380
   Pro Gly Ala Ala Thr Ala Asn Arg Thr Leu Val Pro Asp Ala Thr Asp
   385                 390                 395                 400
<210> 2
   <211> 1203
   <212> DNA
   <213> Rat
<400> 2
   atggagtccg ggctactgcg gccagcgccg gtgagcgagg tcatcgtcct tcactacaac 60
   tacactggca agctccgggg agcgcgctac cagcccggtg ccggcctgcg tgcggacgcc 120
   gcagtgtgct tggctgtgtg cgctttcatc gtgctggaga acctggctgt gctcttggtg 180
   ctgggccgcc atcctcgctt ccatgcaccc atgttcctgc tcctgggtag tcttaccttg 240
   tcggacctgc tcgctggggc ggcctacgcc accaacatcc tgctgtccgg gccgctcaca 300
   ttgcgcctgt cgcctgcgct ctggtttgcg cgcgaagggg gtgtcttcgt ggcgctcgca 360
   gcgtcggtgc tgagcctcct ggccattgct ctagagcgcc accttaccat ggcccgtcgt 420
   ggacccgcgc ctgccgccag tcgcgctcgc acgctggcga tggcggtggc cgcctggggc 480
   ttgtcgctgc tgctggggct gctgcccgcg ctgggctgga actgcttggg acgcctggaa 540
   gcctgctcca ccgtgctgcc gctctacgcc aaggcctatg tgctcttctg cgtgctggcc 600
   ttcctgggca tcctggctgc catctgtgcg ctctatgcaa ggatttactg tcaggtgcgg 660
   gccaacgcgc gtcgcctgcg ggccggtcct gggtcccgta gggccacgtc ctcctcgcga 720
   tcccggcaca cgccacggtc gttggccctg ctccgcacgc ttagcgtggt gctcctggcc 780
   ttcgtggcct gctggggacc tctatttctc ttgctattac tggatgtcgc gtgcccagcc 840
   cgcgcgtgtc ctgtgcttct gcaagccgat cccttcctgg gtctagccat ggctaactcg 900
   ctgctgaatc ctatcatcta caccttcacc aaccgagacc tgcgccacgc gctcctgagg 960
   ctgctctgct gtggccgcgg accctgcaac caagactcct ccaacagttt gcagcgatcc 1020
   ccaagtgctg ttgggccttc cggtggaggc ctgcgacgct gcctgccacc aaccctggat 1080
   cgcagctcta gcccatcaga acactcgtgt ccccagcggg acgggatgga caccagctgc 1140
   tccactggca gtcccggagc agcaaccgcc aaccggaccc tggtgcctga tgctacagac 1200
   tga                                                               1203
<210> 3
   <211> 22
   <212> DNA
   <213> PCR primer
<400> 3
   atttaggtga cactatagaa ta 22
<210> 4
   <211> 19
   <212> DNA
   <213> PCR primer
<400> 4
   gctgcggaag gagtagatg 19
<210> 5
   <211> 20
   <212> DNA
   <213> PCR primer
<400> 5
   cctctagatg catgctcgag 20
<210> 6
   <211> 19
   <212> DNA
   <213> PCR primer
<400> 6
   caggagcagg ccaaacagg 19

## Description

### Field of the Invention

The present invention relates to nucleic acids encoding a novel G protein-coupled receptor and to the receptor itself.

### Background of the Invention

G protein-coupled receptors (GPCRs) constitute a family of proteins sharing a common structural organization characterized by an extracellular N-terminal end, seven hydrophobic alpha helices putatively constituting transmembrane domains and an intracellular C-terminal domain. GPCRs bind a wide variety of ligands that trigger intracellular signals through the activation of transducing G. proteins *(*Caron et al., Rec. Prog. Horm. Res. 48:277-290 (1993); Freedman et al., Rec. Prog. Horm. Res. 51:319-353 (1996)).

Approximately 50-60% of all clinically relevant drugs act by modulating the functions of various GPCRs (Cudermann et al., J. Mol. Med 73:51-63 (1995)). Of particular interest are receptors located in the central nervous system. G protein-coupled receptors in this region are known to be involved in the transmission, modulation and sensation of pain. Thus, G protein-coupled receptors derived from the brain and spinal column may be used in assays for the identification of new anesthetic and analgesic agents.

### Summary of the Invention

The present invention is based upon the discovery of a novel G protein-coupled receptor that is structurally distinct from previously reported receptors. It is referred to herein as the "B1C3 receptor."

In its first aspect, the invention is directed to a protein, except as existing in nature, comprising the amino acid sequence of SEQ ID NO: 1. The term "consisting functionally of" refers to proteins in which the sequence of SEQ ID NO:1 has undergone additions, deletions or substitutions which do not substantially alter the functional characteristics of the receptor. The term is intended to encompass proteins having exactly the same amino acid sequence as that of SEQ ID NO: 1, as well as proteins with sequence differences that are not substantial as evidenced by their retaining the basic, qualitative ligand binding and physiological properties of the B1C3 receptor. The term "except as existing in nature" refers to a compound that is either expressed by recombinant means or that is in a purified (preferably substantially purified) state.

In a preferred embodiment the protein has an amino acid sequence consisting of the sequence of SEQ ID NO: 1. The invention includes antibodies that bind preferentially to such a protein *(i.e.,* antibodies having at least a 100-fold greater affinity for B1C3 than any other protein); and antibodies made by a process involving the injection of a pharmaceutically acceptable preparation of B1C3 into an animal capable of antibody production. Preferably, monoclonal antibody to B1C3 is produced by administering B1C3 to a mouse and then fusing the mouse's spleen cells with myeloma cells.

The invention is also directed to a polynucleotide, except as existing in nature, encoding a protein comprising the amino acid sequence of SEQ ID NO: 1. This aspect of the invention encompasses polynucleotides encoding proteins consisting of the amino acid sequence of SEQ ID NO:1, expression vectors comprising such polynucleotides, and isolated host cells transformed with such vectors. Also included is recombinant B1C3 receptor produced by isolated host cells made in this manner. Preferably, the polynucleotide encoding the B1C3 receptor has a sequence consisting of the nucleotide sequence of SEQ ID NO:2, and the vectors and isolated host cells used for expression of the receptor also use this particular polynucleotide.

In another aspect, the present invention is directed to a method for assaying a test compound for its ability to bind to the B1C3 receptor. The method is performed by incubating a source of B1C3 with a ligand known to bind to the receptor and with the test compound. The source of receptor should, preferably, express a large amount of B1C3 relative to other G protein-coupled receptors. Upon completion of incubation, the ability of the test compound to bind to B1C3 is determined by the extent to which ligand binding has been displaced. Preferably, the receptor present should have the sequence shown in SEQ ID NO:1. Although not essential, the binding assay can be accompanied by an assay to determine whether a second messenger pathway, e.g., the adenyl cyclase pathway, has become activated. This should help to determine whether a particular compound binding to B1C3 is acting as an agonist or antagonist.

A second method for determining if a test compound is an B1C3 agonist, a method that does not require any ligand, is to use a cell signaling assay, e.g., an assay measuring either intracellular adenyl cyclase activity or intracellular calcium concentration. The test compound should generally be incubated with cells expressing high amounts of B1C3 relative to other G protein-coupled receptors, typically a cell transfected with an expression vector encoding the B1C3 of SEQ ID NO:1. Test compounds that are agonists are identified by their causing a statistically significant change in the results obtained from the cell signaling assay when compared to control cells not exposed to test compound. The control cells may be either cells that have not been transfected or cells that have been mock transfected with a vector that does not produce active receptor. B1C3-expressing cells exposed to test compounds that are agonists would typically be expected to show a significant increase in adenyl cyclase activity or in intracellular calcium concentration relative to control cells.

It is a well known that, upon stimulation with an agonist, GPCRs internalize. Thus, another method that can be used to identify an endogenous ligand of B1C3, or to screen for compounds that act as agonists of the receptor, is to determine whether a cell expressing the receptor internalizes it in response to contact with one or more test compounds. One method of accomplishing this is to label the B1C3 receptor with a fluorescent probe (*e.g.,* by expressing it as a fusion protein linked to the green fluorescent protein) and use microscopy to determine whether internalization occurs (see Example 3). In doing this, one may start with a crude extract of compounds and, if internalization occurs, purify the factors responsible using standard methods of biochemistry.

The invention also encompasses a method for determining if a test compound is an antagonist or inverse agonist of B1C3 which relies upon the known constitutive activation of G protein-coupled receptors that occurs when such receptors are expressed in large amounts. This method requires that DNA encoding the receptor be incorporated into an expression vector so that it is operably linked to a promoter and that the vector then be used to transfect an appropriate host. In order to produce sufficient receptor to result in constitutive receptor activation (*i.e.,* activation in the absence of natural ligand), expression systems capable of copious protein production are preferred, e.g., the B1C3 DNA may be operably linked to a CMV promoter and expressed in COS or HEK293 cells. After transfection, cells with activated receptors are selected based upon their showing increased activity in a cell signaling assay relative to comparable cells that have either not been transfected or that have been transfected with a vector that is incapable of expressing functional B1C3. Typically, cells will be selected either because they show a statistically significant change in intracellular adenyl cyclase activity, in intracellular calcium concentration, or in repoter gene activity such as SEAP (secreted alkaline phosphatase). The selected cells are contacted with the test compound and the cell signaling assay is repeated to determine if this results in a decrease in activity relative to selected cells that have not been contacted with the test compound. For example, a statistically significant decrease in either adenyl cyclase activity, calcium concentration or SEAP actvity relative to control cells would indicate that the test compound is an antagonist of B1C3. Preferably the B1C3 used in assays has the sequence of SEQ ID NO:1.

Assays for compounds interacting with B1C3 may be performed by incubating a source containing the receptor (e.g., a stably transformed cell) with a ligand specific for B1C3 both in the presence and absence of test compound and measuring the modulation of intracellular calcium concentration. A significant increase or decrease in ligand-stimulated calcium signaling in response to test compound is indicative of an interaction occurring at the B1C3 receptor. The preferred receptor is that having the amino acid sequence of SEQ ID NO: 1.

In another aspect, the present invention is directed to a method for assaying a test compound for its ability to alter the expression of B1C3. This method is performed by growing cells expressing B1C3 in the presence of the test compound. Cells are then collected and the expression of B1C3 is compared with expression in control cells grown under essentially identical conditions but in the absence of test compound. The preferred receptor is one having the amino acid sequence of SEQ ID NO: 1. A preferred test compound is an oligonucleotide at least 15 nucleotides in length comprising a sequence complementary to the sequence of the B1C3 mRNA used in the assay.

### Brief Description of the Drawings

Figure 1. Figure 1 contains the complete nucleotide sequence of a clone constructed by the methods described in the Examples section. The clone was deposited with the International Depository Authority Deutsche Sammlung Von Mikroorganismen Und Zellkulturen GmbH at the address Mascheroder Weg I B, D-3300 Braunschweig, Germany. The deposit was made on May 14,1999 and was given the accession number DSM 12808.
Figure 2. Figure 2 shows the deduced amino acid sequence of rat B1C3. The polynucleotide of Figure 1 codes for a protein 400 amino acids in length.
Figure 3. Figure 3 shows a sequence comparison between B1C3 and sequences for mouse and rat EDG-1 receptors. B1C3 is about 34% homologous to the mouse EDG-1 receptor and about 33% homologous to the rat EDG-1 receptor.

### Definitions

The description that follows uses a number of terms that refer to recombinant DNA technology. In order to provide a clear and consistent understanding of the specification and claims, including the scope to be given such terms, the following definitions are provided.

Cloning vector: A plasmid or phage DNA or other DNA sequence which is able to replicate autonomously in a host cell and which is characterized by one or a small number of restriction endonuclease recognition sites. A foreign DNA fragment may be spliced into the vector at these sites in order to bring about the replication and cloning of the fragment. The vector may contain a marker suitable for use in the identification of transformed cells. For example, a marker may provide tetracycline resistance or ampicillin resistance.

Expression vector: A vector similar to a cloning vector but which is capable of inducing the expression of the DNA that has been cloned into it, after transformation into a host. The cloned DNA is usually placed under the control of (*i.e*., operably linked to) certain regulatory sequences such as promoters or enhancers. Promoters may be constitutive, inducible or repressible.

Substantially pure: As used herein, "substantially pure" means that the desired product is essentially free from contaminating cellular components. A "substantially pure" protein or nucleic acid will typically comprise at least 85% of a sample, with greater percentages being preferred. Contaminants may include proteins, carbohydrates or lipids. One method for determining the purity of a protein or nucleic acid is by electrophoresing a preparation in a matrix such as polyacrylamide or agarose. Purity is evidenced by the appearance of a single band after staining. Other methods for assessing purity include chromatography and analytical centrifugation.

Recombinant protein: A recombinant protein or recombinant receptor is a non-endogenous protein produced by the introduction of an expression vector into host cells.

Host: Any prokaryotic or eukaryotic cell that is the recipient of a replicable expression vector or cloning vector is the "host" for that vector. The term encompasses prokaryotic or eukaryotic cells that have been engineered to incorporate a desired gene on its chromosome or in its genome. Examples of cells that can serve as hosts are well known in the art, as are techniques for cellular transformation *(see, e.g.,* Sambrook et al., Molecular Cloning: A Laboratory Manual, 2nd ed. Cold Spring Harbor (1989)).

Promoter: A DNA sequence typically found in the 5' region of a gene, located proximal to the start codon. Transcription is initiated at the promoter. If the promoter is of the inducible type, then the rate of transcription increases in response to an inducing agent.

Complementary Nucleotide Sequence: A complementary nucleotide sequence, as used herein, refers to the sequence that would arise by normal base pairing. For example, the nucleotide sequence 5'-AGAC-3'would have the complementary sequence 5'- GTCT-3'.

Expression: Expression is the process by which a polypeptide is produced from DNA. The process involves the transcription of the gene into mRNA and the translation of this mRNA into a polypeptide.

### Detailed Description of the Invention

The present invention is directed to an R1C3 receptor protein, genetic sequences coding for the protein, methods for assaying compounds to determine if they interact with B1C3 and a method for assaying compounds for their ability to alter receptor expression.

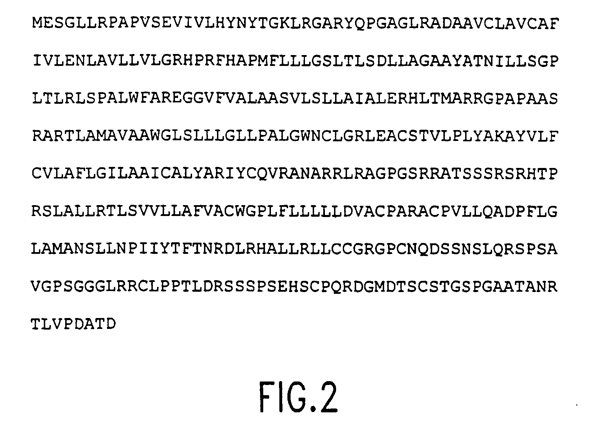

The nucleic acid encoding the receptor and the receptor itself are defined by the structures shown in Figure I (SEQ ID NO:2) and Figure 2 (SEQ ID NO: 1) respectively. However, the invention encompasses not only sequences identical to those shown in the figures and sequence listing, but also sequences that are essentially the same and sequences that are otherwise substantially the same and which result in a receptor retaining the basic binding characteristics of B1C3. For example, it is well known that techniques such as site-directed mutagenesis may be used to introduce variations into a protein's structure. Variations in the B1C3 receptor introduced by this or some similar method are encompassed by the invention provided that the resulting receptor retains the basic qualitative binding and physiological characteristics of unaltered B1C3. Thus, the invention relates to proteins comprising amino acid sequences consisting functionally of SEQ ID NO:1.

### I. Nucleic Acid Sequences Coding for B1C3

DNA sequences coding for rat B 1 C3 are expressed throughout the adult rat central nervous system. Tissue from these areas may be used as a source for the isolation of nucleic acids coding for the receptor. In addition, cells and cell lines that express B1C3 may be used. These may either be cultured cells that have not undergone transformation or cell lines specifically engineered to express recombinant B1C3. Most preferred are the cells deposited as DSMZ No. 12808. DNA encoding B1C3 may be obtained as the result of standard restriction digestions. Alternatively, poly A⁺mRNA may be isolated from tissue or cells, reverse transcribed and cloned. The cDNA library thus formed may then be screened using probes derived from SEQ ID NO:2. Probes should typically be at least 14 bases in length and should, preferably, not be obtained from regions of the DNA corresponding to highly conserved transmembrane domains of B 1 C3.

B1C3 can also be obtained from recombinant cells containing the full-length B 1 C3 sequence or from cDNA libraries by performing PCR amplifications using primers located at either end of the B 1C3 gene. These primers can be selected from the sequences shown in SEQ ID NO:2.

### II. Antibodies to B1C3

The present invention is also directed to antibodies that bind specifically to B1C3 and to a process for producing such antibodies. Antibodies that "bind specifically" are defined as those that have at least a one hundred fold greater affinity for B1C3 than for other proteins. The process for producing such antibodies may involve either injecting the B1C3 protein itself into an appropriate animal or, alternatively, injecting short peptides made to correspond to different regions of the receptor. The peptides should be at least five amino acids in length and should be selected from regions believed to be unique to B1C3. Thus, highly conserved transmembrane regions should generally be avoided in selecting peptides for the generation of antibodies. Methods for making and detecting antibodies are well known to those of skill in the art as evidenced by standard reference works such as: Harlow et al., Antibodies. A Laboratory Manual, Cold Spring Harbor Laboratory, N.Y. (1988)); Klein, Immunology: The Science of Self-Nonself Discrimination (1982); Kennett et al., Monoclonal Antibodies and Hybridomas: A New Dimension in Biological Analyses (1980); and Campbell, "Monoclonal Antibody Technology," in Laboratory Techniques in Biochemistry and Molecular Biology, (1984)).

"Antibody," as used herein, is meant to include intact molecules as well as fragments which retain their ability to bind to antigen (*e.g*., Fab and F(ab')₂ fragments). These fragments are typically produced by proteolytically cleaving intact antibodies using enzymes such as papain (to produce Fab fragments) or pepsin (to produce F(ab')₂ fragments). The term "antibody" also refers to both monoclonal antibodies and polyclonal antibodies. Polyclonal antibodies are derived from the sera of animals immunized with the antigen. Monoclonal antibodies can be prepared using hybridoma technology (Kohler et al., Nature 256:495 (1975); Hammerling et al., in: Monoclonal Antibodies and T-Cell Hybridomas, Elsevier, M.Y., pp. 563-681 (1981)). In general, this technology involves immunizing an animal, usually a mouse, with either intact B 1 C3 or a fragment derived from B1C3. The splenocytes of the immunized animals are extracted and fused with suitable myeloma cells, *e.g*., SP₂O cells. After fusion, the resulting hybridoma cells are selectively maintained in HAT medium and then cloned by limiting dilution (Wands et al., Gastroenterology 80:225-232 (1981)). The cells obtained through such selection are then assayed to identify clones which secrete antibodies capable of binding to B1C3.

The antibodies, or fragments of antibodies, of the present invention may be used to detect the presence of B 1C3 using any of a variety of immunoassays. For example, the antibodies may be used in radioimmunoassays or in immunometric assays, also known as "two-site" or "sandwich" assays (*see* Chard, T., "An Introduction to Radioimmune Assay and Related Techniques," in Laboratory Techniques in Biochemistry and Molecular Biology, North Holland Publishing Co., N.Y. (1978)). In a typical immunometric assay, a quantity of unlabeled antibody is bound to a solid support that is insoluble in the fluid being tested, e.g., blood, lymph, cellular extracts, etc. After the initial binding of antigen to immobilized antibody, a quantity of detectably labeled second antibody (which may or may not be the same as the first) is added to permit detection and/or quantitation of bound antigen (*see, e.g.,* Radioimmune Assay Method, Kirkham et al., ed., pp. 199-206, E & S. Livingstone, Edinburgh (1970)). Many variations of these types of assays are known in the art and may be employed for the detection of B1C3.

Antibodies to B1C3 may also be used in the purification of either intact receptor or fragments of the receptor *(see generally,* Dean et al., Affinity Chromatography. A Practical Approach, IRL Press (1986)). Typically, antibody is immobilized on a chromatographic matrix such as Sepharose 4B. The matrix is then packed into a column and the preparation containing B1C3 is passed through under conditions that promote binding, e.g., under conditions of low salt. The column is then washed and bound B 1C3 is eluted using a buffer that promotes dissociation from antibody, e.g., buffer having an altered pH or salt concentration. The eluted B1C3 may be transferred into a buffer of choice, e.g., by dialysis, and either stored or used directly.

### III. Radioligand Assay for Receptor Binding

One of the main uses for B1C3 nucleic acids and recombinant proteins is in assays designed to identify agents capable of binding to the receptor. Such agents may either be agonists, mimicking the normal effects of receptor binding, or antagonists, inhibiting the normal effects of receptor binding. Of particular interest is the identification of agents which bind to the B1C3 receptor and modulate intracellular signaling, such as adenyl cyclase activity or intracellular calcium. These agents have potential therapeutic application as either analgesics or anesthetics.

In radioligand binding assays, a source of B1C3 is incubated together with a ligand known to bind to the receptor and with the compound being tested for binding activity. The preferred source of B1C3 is cells, preferably mammalian cells, transformed to recombinantly express the receptor. The cells selected should not express a substantial amount of any other G protein-coupled receptor that might bind to ligand and distort results. This can easily be determined by performing binding assays on cells derived from the same tissue or cell line as those recombinantly expressing B1C3 but which have not undergone transformation.

The assay may be performed either with intact cells or with membranes prepared from the cells (*see, e.g.,* Wang et al., Proc. Natl. Acad. Sci. U.S.A. 90:10230-10234 (1993)). The membranes, or cells, are incubated with a ligand specific for the B1C3 receptor and with a preparation of the compound being tested. After binding is complete, receptor is separated from the solution containing ligand and test compound, e.g., by filtration, and the amount of binding that has occurred is determined. Preferably, the ligand used is detectably labeled with a radioisotope such as ¹²⁵I. However, if desired, fluorescent or chemiluminescent labels can be used instead. Among the most commonly used fluorescent labeling compounds are fluorescein isothiocynate, rhodamine, phycoerythrin, phycocyanin, allophycocyanin, o-phthaldehyde and fluorescamine. Useful chemiluminescent compounds include luminol, isoluminol, theromatic acridinium ester, imidazole, acridinium salt, and oxalate ester. Any of these agents can be used to produce a ligand suitable for use in the assay.

Nonspecific binding may be determined by carrying out the binding reaction in the presence of a large excess of unlabeled ligand. For example, labeled ligand may be incubated with receptor and test compound in the presence of a thousandfold excess of unlabeled ligand. Nonspecific binding should be subtracted from total binding, i.e. binding in the absence of unlabeled ligand, to arrive at the specific binding for each sample tested. Other steps such as washing, stirring, shaking, filtering and the like may be included in the assays as necessary. Typically, wash steps are included after the separation of membrane-bound ligand from ligand remaining in solution and prior to quantitation of the amount of ligand bound, e.g., by counting radioactive isotope. The specific binding obtained in the presence of test compound is compared with that obtained in the presence of labeled ligand alone to determine the extent to which the test compound has displaced receptor binding.

In performing binding assays, care must be taken to avoid artifacts which may make it appear that a test compound is interacting with the B1C3 receptor when, in fact, binding is being inhibited by some other mechanism. For example, the compound being tested should be in a buffer which does not itself substantially inhibit the binding of ligand to B1C3 and should, preferably, be tested at several different concentrations. Preparations of test compound should also be examined for proteolytic activity and it is desirable that antiproteases be included in assays. Finally, it is highly desirable that compounds identified as displacing the binding of ligand to B1C3 receptor be reexamined in a concentration range sufficient to perform a Scatchard analysis of the results. This type of analysis is well known in the art and can be used for determining the affinity of a test compounds for receptor *(see, e.g.,* Ausubel et al., Current Protocols in Molecular Biology, 11.2.1-11.2.19 (1993); Laboratory Techniques in Biochemistry and Molecular Biology, Work et al., ed., N.Y. (1978) etc.). Computer programs may be used to help in the analysis of results *(see, e.g.,* Munson, P., Methods Enzymol. 92:543-577 (1983); McPherson, G.A., Kinetic, EBDA Ligand, Lowry-A Collection of Radioligand Binding Analysis Programs, Elsevier-Biosoft, U.K. (1985)).

The activation of receptor by the binding of ligand may be monitored using a number of different assays. For example, adenyl cyclase assays may be performed by growing cells in wells of a microtiter plate and then incubating the wells in the presence or absence of test compound. cAMP may then be extracted in ethanol, lyophilized and resuspended in assay buffer. Assay of cAMP thus recovered may be carried out using any method for determining cAMP concentration, e.g. the Biotrack cAMP Enzyme-immunoassay SystemJ (Amersham) or the Cyclic AMP [³H] Assay System (Amersham). Typically, adenyl cyclase assays will be performed separately from binding assays, but it may also be possible to perform binding and adenyl cyclase assays on a single preparation of cells. Other "cell signaling assays" that can be used to monitor receptor activity are described below.

### IV. Identification of B1C3 Agonists and Antagonists Using Cell Signaling Assays

B 1C3 receptors may also be used to screen for drug candidates using cell signaling assays. To identify B1C3 agonists, the DNA encoding a receptor is incorporated into an expression vector and then transfected into an appropriate host. The transformed cells are then contacted with a series of test compounds and the effect of each is monitored. Among the assays that can be used are assays measuring cAMP production (see discussion above), assays measuring the activation of reporter gene activity, assays measuring the modulation of the binding of ligand, e.g., GTP-gamma-S, or assays measuring changes in intracellular calcium concentration.

Cell signaling assays may also be used to identify B1C3 antagonists. G protein-coupled receptors can be put into their active state even in the absence of their cognate ligand by expressing them at very high concentration in a heterologous system. For example, receptor may be overexpressed using the baculovirus infection of insect Sf9 cells or the B1C3 gene may be operably linked to a CMV promoter and expressed in COS or HEK293 cells. In this activated constitutive state, antagonists of the receptor can be identified in the absence of ligand by measuring the ability of a test compound to inhibit constitutive cell signaling activity. Appropriate assays for this are, again, cAMP assays, reporter gene activation assays or assays measuring the binding of GTP-gamma-S.

One preferred cell signaling assay is based upon cells stably transfected with B1C3 showing a change in intracellular calcium levels in response to incubation in the presence of ligand. Thus, a procedure can be used to identify B1C3 agonists or antagonists that is similar to the radioreceptor assays discussed above except that calcium concentration is measured instead of bound radioactivity. The concentration of calcium in the presence of test compound and ligand is compared with that in the presence of ligand alone to determine whether the test compound is interacting at the B1C3 receptor. A statistically significant increase in intracellular calcium in response to test compound indicates that the test compound is acting as an agonist whereas a statistically significant decrease in intracellular calcium indicates that it is acting as an antagonist.

Assays may also be performed that measure the activation of a reporter gene. For example, cells expressing recombinant B1C3 may be transfected with a reporter gene (e.g., a chloramphenicol acetyltransferase or luciferase gene) operably linked to an adenyl cyclase or diacylglycerol response element. The cells are then incubated with test compounds and the expression of the reporter gene is compared to expression in control cells that do not express recombinant B 1C3 but that are essentially identical in other respects. A statistically significant change in reporter gene expression in the B1C3-expressing cells is indicative of a test compound that interacts with the B1C3 receptor.

### V. Assay for Ability to Modulate B1C3 Expression

One way to either increase or decrease the biological effects of B1C3 is to alter the extent to which the receptor is expressed in cells. Therefore, assays for the identification of compounds that either inhibit or enhance expression are of considerable interest. These assays are carried out by growing cells expressing B1C3 in the presence of a test compound and then comparing receptor expression in these cells with expression in cells grown under essentially identical conditions but in the absence of test compound. As in the binding assays discussed above, it is desirable that the cells used be substantially free of competing G protein-coupled receptors. One way to measure receptor expression is to fuse the B1C3 sequence to a sequence encoding a peptide or protein that can be readily quantitated. For example, the B1C3 sequence may be ligated to a sequence encoding hemagglutinin and used to stably transfect cells. After incubation with test compound, the hemagglutinin/receptor complex can be immuno-precipitated and Western blots performed using anti-hemagglutinin antibody. Alternatively, Scatchard analysis of binding assays may be performed with labeled ligand to determine receptor number. The binding assays may be carried out as discussed above and will preferably utilize cells that have been engineered to recombinantly express B1C3.

A preferred group of test compounds for inclusion in the B 1C3 expression assay consists of oligonucleotides complementary to various segments of the B1C3 nucleic acid sequence shown in SEQ ID NO:2. These oligonucleotides should be at least 15 bases in length and should be derived from non-conserved regions of the receptor nucleic acid sequence.

Oligonucleotides which are found to reduce receptor expression may be derivatized or conjugated in order to increase their effectiveness. For example, nucleoside phosphorothioates may be substituted for their natural counterparts *(see* Cohen, J., Oligodeoxynucleotides, Antisense Inhibitors of Gene Expression, CRC Press (1989)). The oligonucleotides may also be delivered to a patient *in vivo* for the purpose of inhibiting B1C3 expression. When this is done, it is preferred that the oligonucleotide be administered in a form that enhances its uptake by cells. For example, the oligonucleotide may be delivered by means of a liposome or conjugated to a peptide that is ingested by cells *(see, e.g.,* U.S. Patent Nos. 4,897,355 and 4,394,448; *see also* non-U.S. patent documents WO 8903849 and EP 0263740). Other methods for enhancing the efficiency of oligonucleotide delivery are well known in the art and are also compatible with the present invention.

Having now described the invention, the same will be more readily understood through reference to the following Examples which are provided by way of illustration and which are not intended to limit the scope of the invention.

### Examples

### Example 1: Cloning of Rat BC13

*Generation of Probe for cDNA Library Screening:* One method of obtaining a novel sequence encoding a receptor is to perform moderate to low stringency screening of cDNA libraries with a relevant probe. In order to find novel genes related to receptors for lipids (sphingosine phosphate, lysophosphatidic acid, cannabinoids etc) the sequence of an EST (Expressed Sequence Tag), aa451451, was used to generate a cDNA probe for the screening cDNA libraries. The following oligonucleotides were used for PCR:

| | |
|---|---|
| 5' ATT TAG GTG ACA CTA TAG AAT A 3' | SP6 primer (SEQ ID NO:3) |
| 5' GCT GCG GAA GGA GTA GAT G 3' | 451-1R primer (SEQ ID NO:4) |
| 5' CCT CTA GAT GCA TGC TCG AG 3' | SP6-1R internal primer (SEQ IDNO:5) |
| 5' CAG GAG CAG GCC AAA CAG G 3' | 451-3R internal primer (SEQ ID NO:6) |

Two of these oligonucleotides were used in a polymerase chain reaction mixture (total volume 100 µl), which contained 1.5il of a rat brain (minus cerebellum) cDNA library in pcDNA1 (Invitrogen), 1X PCR buffer (14 mM (NH₄)₂SO₄, 1.5 mM MgCl₂, 50 mM Tris-HCl (pH 9.2), Roche Diagnostics), 200 µM dNTPs (Amersham-Pharmacia), 2.6U Expand High FidelityJ (Roche Diagnostics) and 100 pmoles each of the SP6 and 451-1R primers. Amplification was carried out on a Gene Amp PCR Sys 9700J (Perkin Elmer: Applied Biosystems). The template was denatured at 94°C for 3 minutes, followed by 35 cycles consisting of denaturation, annealing and extension steps, each for 1 minute at 94°C, 53°C and 72°C, respectively, and then extended for an additional 5 minutes at 72°C.

The resulting product was diluted 1:100 and amplified with primers SP6-1R and 451-3R. The polymerase chain reaction mixture (50il) contained 1 il of diluted solution from the first PCR reaction, 1X PCR buffer (same as above, Roche Diagnostics), 200iM dNTPs (Amersham-Pharmacia), 2.6U Expand High FidelityJ (Roche Diagnostics) and 50 pmoles each of the SP6-1R and 451-3R primers. The amplification was carried out on a Gene Amp PCR Sys 9700J (Perkin Elmer: Applied Biosystems). Template was denatured at 94°C for 3 minutes, followed by 25 cycles consisting of denaturation, annealing and extension steps at 94°C (1 minute), 53°C (1 minute) and 72°C (1 minute 20 seconds) respectively, and then extended for an additional 5 minutes at 72°C.

A PCR product of 450 bp was excised and purified with QIAquickJ gel extraction kit (Qiagen), blunted with Klenow (Amersham-Pharmacia) and subcloned into KS- digested by ER V and dephosphorylated. The plasmids were prepared with the alkaline lysis protocol using a Qiaprep 8J kit (Qiagen) and screened by sequencing using ABI Prism dRhodamineJ cycle sequencing ready reaction kit (PE Applied Biosystems). The sequence of the 450bp rat fragment was identical to the of EST aa451451. A NotI/HindIII fragment of 400bp was then digested, excised and purified with QlAquickJ gel extraction kit (Qiagen) to be used as a tool to isolate an EDG related rat clone.

cDNA Library Screening: A Stratagene rat brainstem/spinal cord cDNA library (cat # 936521) was screened using the NotI/HindIII fragment as a probe. 8X10⁵ plaques were plated, transferred to Hybond N+J filters (Amersham-Pharmacia), denatured for 5 minutes (1.5M NaCl and 0.5M NaOH) and neutralized for 5 minutes (1.5M NaCl, 0.5M Tris-HCl pH 8). The filters were rinsed in 2X SSC and air-dried for one hour at room temperature. The membranes were hybridized overnight at 42°C in 50% formamide, 10% dextran sulphate (from a 50% solution), 1% SDS, 5XSSC, 1X Denhardt's, 10mM Tris pH8 and 100_g/ml of sonicated salmon sperm. The Notl/Hindlll fragment was randomly labeled using T7 Quick PrimeJ (Amersham-Pharmacia) and added to the hybridization solution at 3X10⁶cpm/ml. The filters were washed with 2XSSC/ 0.1%SDS at room temperature followed by a high stringency wash with 0.2XSSC/ 0.1 % SDS at 65°C.

Using this screening procedure, 6 independent clones were identified and isolated by repeated plating and screening with the labeled NotI/HindIII fragment. Two independent clones were subjected to sequencing (clones BlA7 and C3A3). The DNA sequence analysis revealed that the clone B1A7 contained the 5' sequence of a putative novel GPCR but was missing the 3' end. Although C3A3 contained the whole coding sequence, it was missing one nucleotide, leading to a premature stop codon. In order to obtain the full coding sequence, the 2 clones were digested by XbaI and ligated together. The resulting clone "B 1C3" contains the complete coding region of a putative novel GPCR.

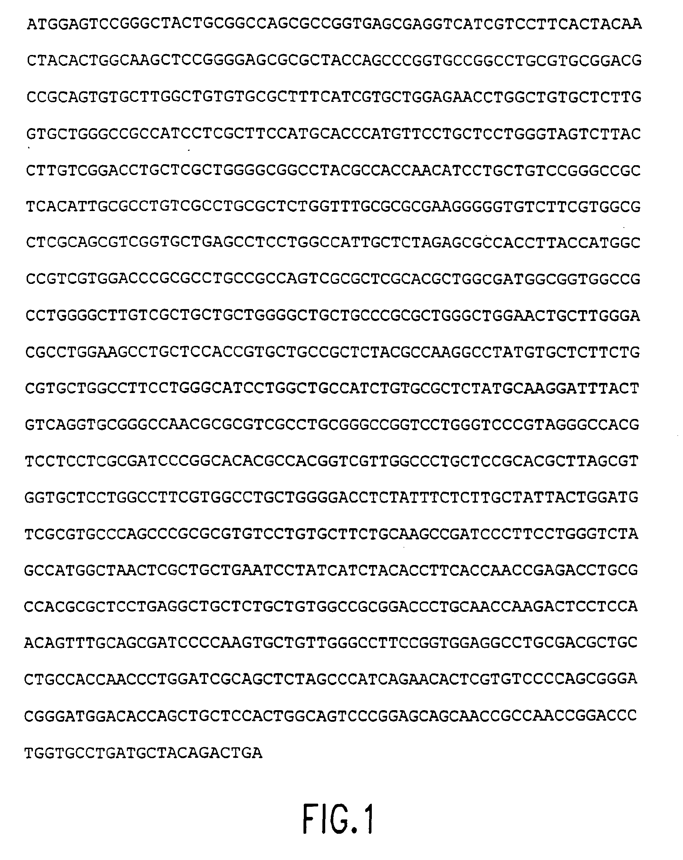

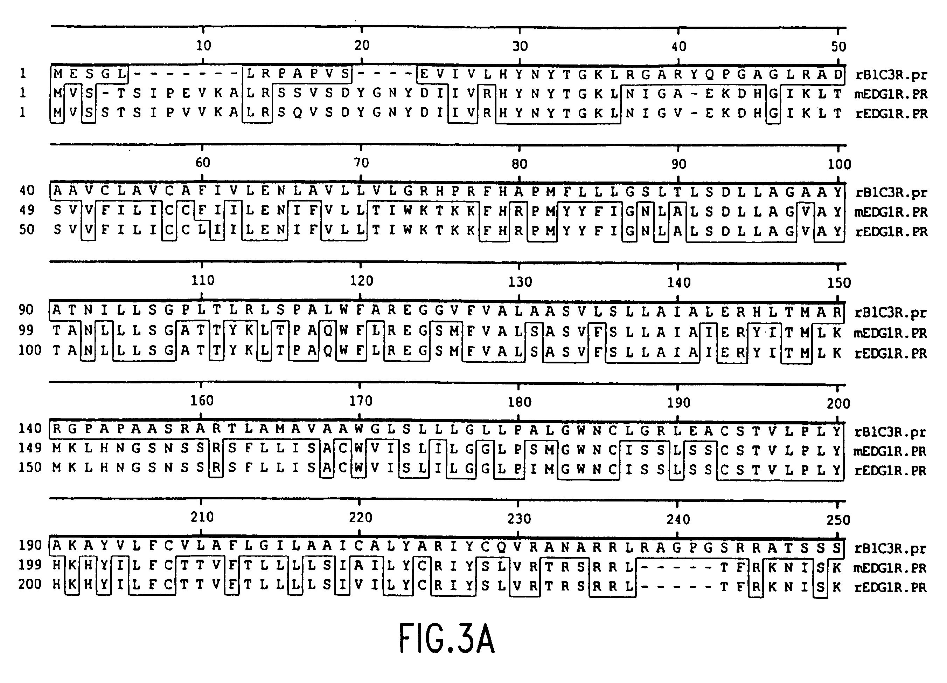

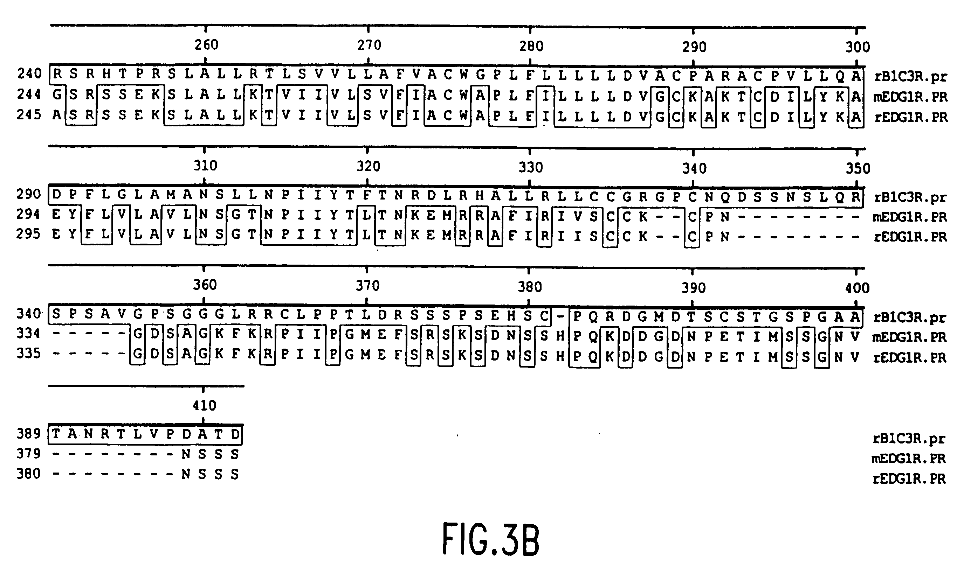

*Results:* The complete nucleotide sequence of the B1C3 cDNA clone is shown in Figure 1. The open reading frame is comprised of 1203 nucleotides, encoding a protein of 401 amino acids (Figure 2) with a predicted molecular mass of about 42.3 kDa. The protein sequence contains hallmark features of GPCRs: the presence of seven hydrophobic helices likely to represent transmembrane domains; an amino terminus; and a carboxy terminus. In addition, several modification sites proposed to be involved in the regulation of receptor function are also present in the predicted amino acid sequence. Thus, the receptor sequence has: a potential cAMP phosphorylation site at position 236; N-linked glycosylation sites; myristilation sites; and Protein Kinase-C phosphorylation sites. The nucleotide sequence and primary predicted amino acid sequence of the B1C3 receptor are not present in their entirety in the Genebank database. The sequences most closely resemble those of the mouse and rat EDG-1 receptor (epithelial differentiation gene receptor). A sequence alignment of B1C3 with known receptors reveals that it is about 34 % identical to mouse EDG-1 and 33% identical to rat EDG-1 receptors (Figure 3).

### Example 2: In Situ Hybridization Experiments

*Preparation of tissue:* Adult male Sprague-Dawley rats (about 250 gm; Charles River, St.-Constant, Quebec) were sacrificed by decapitation. Brain, spinal cord with DRGs still attached and several peripheral tissues (heart, kidney, spleen, liver, lung and skeletal muscle) were promptly removed, snap-frozen in isopentane at -40°C for 20 sec. and stored at -80°C. Frozen tissue was sectioned at 16_m in a Microm HM 500 MJ cryostat (Germany) and thaw-mounted onto ProbeOn PlusJ slides (Fisher Scientific, Montreal, Quebec). Sections were stored at -80°C prior to *in situ* hybridization.

*Riboprobe synthesis:* The pBluescript KS⁻-B1A7 plasmid, containing a 1 Kb fragment of the 5'end of the B1A7 gene, was linearized by cutting in the polylinker site on either side of the inserted cDNA using either NotI or SacI restriction enzymes (Pharmacia Biotech, U.S.A) and the appropriate buffers. Antisense and sense B1A7 riboprobes were transcribed *in vitro* using either T7 or T3 RNA polymerase (Promega), respectively, in the presence of -[³⁵S]-UTP (about 800 Ci/mmol; Amersham, Oakville, Ontario

*In situ Hybridization:* Sections were postfixed in 4% paraformaldehyde (BDH, Poole, England) in 0.1 M phosphate buffer (pH 7.4) for 10 min at room temperature (RT) and rinsed in 3 changes of 2X standard sodium citrate buffer (SSC; 0.15 M NaCl. 0.015 M sodium citrate, pH 7.0). Sections were then equilibrated in 0.1 M triethanolamine, treated with 0.25% acetic anhydride in triethanolamine, rinsed in 2X SSC and dehydrated in an ethanol series (50-100%). Hybridization was performed in a buffer containing 75% formamide (Sigma, St-Louis, Mo.), 600 mM NaCl, 10 mM Tris (pH 7.5), 1 mM EDTA, 1X Denhardt's solution (Sigma), 50 _g/ml denatured salmon sperm DNA (Sigma), 10% dextran sulfate (Sigma), 20 mM dithiothreitol and -[³⁵S]-UTP-labeled cRNA probes (10 X106 cpm/ml) at 55°C for 18 h in humidified chambers. Following hybridization, slides were rinsed in 2X SSC at room temperature, treated with 20 g/ml RNase IA (Pharmacia) in RNase buffer (10 mM Tris, 500 mM NaCl, 1 mM EDTA, pH 7.5) for 45 min at 37° C and washed to a final stringency of 0.1X SSC at 70°C. Sections were then dehydrated with ethanol and exposed to Kodak Biomax MRJ film for 14-21 days and dipped in Kodak NTB2 emulsion diluted 1:1 with distilled water and exposed for 7-8 weeks at 4°C prior to development and counterstaining with cresyl violet acetate (Sigma).

*Results: In situ* hybridization film autoradiograms reveal that B1A7 mRNA is expressed ubiquitously in the adult rat central nervous system (CNS). In the rat spinal cord, the B1A7 mRNA is highly expressed uniformly throughout the white and gray matter. In the rat brain, however, B1A7 mRNA is variably expressed. The receptor's mRNA is primarily expressed in fiber tracts throughout the brain, namely, the corpus callosum, the anterior commissure (anterior and posterior), fornix, fimbria (of the hippocampus), the internal capsule and the cerebellar peduncle. Other structures including the piriform cortex of the olfactory lobe, the hippocampus and the habenular nucleus are moderately labeled; while various other regions of the brain, such as the cortex and the periaqueductal gray, for example, mildly express message. Microscopic examination of emulsion processed sections suggests that these cells are non-neuronal.

In addition to this central distribution, a moderate hybridization signal for B1A7 message is detected in adult rat spleen, but not in heart, kidney, liver, lung or skeletal muscle. Preliminary results, using the rat B1A7 riboprobe, indicate that the B1A7 receptor mRNA is similarly distributed in the mouse CNS, while no labeling is detected in human adult spinal cords or DRGs.

### Example 3: Development of an Assay for the Endogenous Ligand

One method that can be used to identify th<>e endogenous ligand or to screen natural or synthetic compounds is to structurally link the re<>ceptor preferably through its carboxy terminus to a fluorescent probe, for example the green fluorescent protein (GFP). It is a well known fact that upon stimulation with an agonist<>, the GPCRs internalize (Ashworth et al., Proc. Nat. Acad. Sci. USA, 92:512-516 (1995)). When cou<>pled to a fluorescent probe *(e.g.,* GFP) one can easily monitor the internalization of the receptor by conventional techniques (e.g., fluorescent or confocal microscopy, <>etc.). Therefore, one can make the tissue extract from brain, spinal cord etc., incubate a fractionated extract with the cells expressing the hybrid GPCR-GFP construct and monitor internalization. Only the fractions containing endogenous ligand for this particular GPCR will cause the hybrid to internalize. Such an active fraction can then be further fractionated until the recovery of a pure ligand has been achieved. Similarly, one can assay for the receptor activating compounds in a complex mixture of synthetic library of compounds.

## Claims

1. A protein, except as existing in nature, comprising the amino acid sequence of SEQ ID NO:1.

2. The protein of claim 1, wherein said protein consists of the amino acid sequence of SEQ ID NO: 1.

3. An antibody having at least 100-fold greater affinity for the protein of claim 1, or claim 2, than any other protein.

4. A polynucleotide, except as existing in nature, encoding a protein comprising the amino acid sequence of SEQ ID NO: 1.

5. The polynucleotide of claim 4, wherein said polynucleotide encodes a protein consisting of the amino acid sequence of SEQ ID NO:1.

6. The polynucleotide of claim 4, wherein said polynucleotide consists of the nucleotide squence of SEQ ID NO:2.

7. An expression vector comprising the polynucleotide of any one of claims 4-6.

8. An isolated host cell transformed with the vector of claim 7.

9. A method for determining if a test compound is an agonist of the protein of claim 1 or claim 2 comprising:
a) incubating a cell expressing the protein of claim 1 or claim 2 with said test compound; and
b) determining whether said test compound causes a statistically significant increase in either intracellular adenyl cyclase activity or the intracellular concentration of calcium.

10. A method for determining if a test compound is a ligand or agonist of the protein of claim1 or claim 2 comprising determining whether a cell expressing a protein comprising the amino acid sequence consisting functionally of SEQ ID NO:1 internalizes said protein in response to contact with said test compound.

11. The method of claim 10, wherein:
a) said protein is recombinantly expressed in said cell as a fusion protein linked to a fluorescent protein;
b) the cell of step (a) is contacted with said test compound; and
c) internalization of said protein is determined by microscopy.

12. A method for determining if a test compound is an antagonist of the protein of claim 1 or claim 2 comprising:
a) incorporating a DNA molecule encoding the protein of claim 1 or claim 2 into an expression vector so that it is operably linked to a promoter;
b) transfecting said expression vector into a host;
c) selecting cells transfected in step b) that have constitutively activated protein as evidenced by either:
i) a statistically significant increase in intracellular adenyl cyclase activity;or
ii) a statistically significant increase in intracellular calcium concentration;
d) contacting the cells selected in step c) with said test compound; and
e) determining if said test compound causes a statistically significant decrease in either said adenyl cyclase activity or said calcium concentration relative to control cells not contacted with said test compound.

13. A method for assaying a test compound for its ability to alter the expression of the protein of claim 1 or claim 2 comprising:
a) growing cells expressing the protein of claim 1 or claim 2;
b) collecting said cells; and
c) comparing protein expression in the cells exposed to said test compound with control cells grown under essentially identical conditions but not exposed to said test compound,

14. The method of claim 13, wherein said cells expressing said protein are cells transformed with an expression vector comprising a polynucleotide sequence encoding the protein of claim 1 or claim 2.

15. The method of either claim 13 or claim 14, wherein said test compound is an oligonucleotide at least 15 nucleotides in length and comprising a sequence complementary to the polynucleotide sequence encoding said protein.

## Patentansprüche

1. Protein, das nicht in der Natur vorkommt und die Aminosäuresequenz der SEQ ID NO:1 umfaßt.

2. Protein nach Anspruch 1, wobei das Protein aus der Aminosäuresequenz der SEQ ID NO:1 besteht.

3. Antikörper mit einer mindestens 100mal größeren Affinität für das Protein nach Anspruch 1 oder Anspruch 2 als irgendein anderes Protein.

4. Polynukleotid, das nicht in der Natur vorkommt und für ein die Aminosäuresequenz der SEQ ID NO:1 umfassendes Protein codiert.

5. Polynukleotid nach Anspruch 4, wobei das Polynukleotid für ein aus der Aminosäuresequenz der SEQ ID NO:1 bestehendes Protein codiert.

6. Polynukleotid nach Anspruch 4, wobei das Polynukleotid aus der Nukleotidsequenz der SEQ ID NO:2 besteht.

7. Expressionsvektor, der das Polynukleotid nach einem der Ansprüche 4 - 6 umfaßt.

8. Isolierte Wirtszelle, die mit dem Vektor nach Anspruch 7 transformiert ist.

9. Verfahren zur Bestimmung davon, ob es sich bei einer Testverbindung um einen Agonisten des Proteins nach Anspruch 1 oder Anspruch 2 handelt, bei dem man:
a) eine das Protein nach Anspruch 1 oder Anspruch 2 exprimierende Zelle mit der Testverbindung inkubiert und
b) bestimmt, ob die Testverbindung einen statistisch signifikanten Anstieg entweder der intrazellulären Adenylcyclaseaktivität oder der intrazellulären Calciumkonzentration hervorruft.

10. Verfahren zur Bestimmung davon, ob es sich bei einer Testverbindung um einen Liganden oder Agonisten des Proteins nach Anspruch 1 oder Anspruch 2 handelt, bei dem man bestimmt, ob eine ein die funktionell aus der SEQ ID NO:1 bestehende Aminosäuresequenz umfassendes Protein exprimierende Zelle als Antwort auf einen Kontakt mit der Testverbindung das Protein internalisiert.

11. Verfahren nach Anspruch 10, wobei:
a) das Protein in der Zelle als ein mit einem Fluoreszenzprotein verknüpftes Fusionsprotein rekombinant exprimiert wird,
b) die Zelle aus Schritt a) mit der Testverbindung in Kontakt gebracht wird und
c) die Internalisierung des Proteins durch Mikroskopie bestimmt wird.

12. Verfahren zur Bestimmung davon, ob es sich bei einer Testverbindung um einen Antagonisten des Proteins nach Anspruch 1 oder Anspruch 2 handelt, bei dem man:
a) ein für das Protein nach Anspruch 1 oder Anspruch 2 codierendes DNA-Molekül in einen Expressionsvektor einbaut, so daß es mit einem Promotor operativ verknüpft ist,
b) den Expressionsvektor in einen Wirt transfiziert,
c) in Schritt b) transfizierte Zellen, die konstitutiv aktiviertes Protein aufweisen, wie entweder anhand:
i) eines statistisch signifikanten Anstiegs der intrazellulären Adenylcyclaseaktivität oder
ii) eines statistisch signifikanten Anstiegs der intrazellulären Calciumkonzentration zu sehen ist, selektioniert,
d) die in Schritt c) selektionierten Zellen mit der Testverbindung in Kontakt bringt und
e) bestimmt, ob die Testverbindung eine statistisch signifikante Abnahme entweder der Adenylcyclaseaktivität oder der Calciumkonzentration im Vergleich zu nicht mit der Testverbindung in Kontakt gebrachten Zellen hervorruft.

13. Verfahren zum Testen einer Testverbindung auf ihre Fähigkeit zur Veränderung der Expression des Proteins nach Anspruch 1 oder Anspruch 2, bei dem man:
a) das Protein nach Anspruch 1 oder Anspruch 2 exprimierende Zellen kultiviert,
b) die Zellen sammelt und
c) die Proteinexpression in den mit der Testverbindung in Kontakt gekommenen Zellen mit in unter im wesentlichen identischen Bedingungen kultivierten, jedoch nicht mit der Testverbindung in Kontakt gekommenen Zellen vergleicht.

14. Verfahren nach Anspruch 13, wobei es sich bei den das Protein exprimierenden Zellen um mit einem eine für das Protein nach Anspruch 1 oder Anspruch 2 codierende Polynukleotidsequenz umfassenden Vektor transformierte Zellen handelt.

15. Verfahren nach Anspruch 13 oder Anspruch 14, wobei es sich bei der Testverbindung um ein Oligonukleotid mit einer Länge von mindestens 15 Nukleotiden handelt, das eine zu der für das Protein codierenden Polynukleotidsequenz komplementäre Sequenz umfaßt.

## Revendications

1. Protéine, à l'exception d'une existant dans la nature, comprenant la séquence d'acides aminés de SEQ ID NO : 1.

2. Protéine selon la revendication 1, ladite protéine étant constituée de la séquence d'acides aminés de SEQ ID NO : 1.

3. Anticorps ayant une affinité au moins 100 fois plus élevée pour la protéine selon la revendication 1 ou la revendication 2, que pour toute autre protéine.

4. Polynucléotide, à l'exception d'un existant dans la nature, codant pour une protéine comprenant la séquence d'acides aminés de SEQ ID NO . 1.

5. Polynucléotide selon la revendication 4, ledit polynucléotide codant pour une protéine constituée de la séquence d'acides aminés de SEQ ID NO . 1.

6. Polynucléotide selon la revendication 4, ledit polynucléotide étant constitué de la séquence nucléotidique de SEQ ID NO : 2.

7. Vecteur d'expression comprenant le polynucléotide selon l'une quelconque des revendications 4 à 6.

8. Cellule hôte isolée transformée par le vecteur selon la revendication 7.

9. Procédé permettant de déterminer si un composé à l'essai est un agoniste de la protéine selon la revendication 1 ou la revendication 2, comprenant les étapes consistant à :
a) incuber une cellule exprimant la protéine selon la revendication 1 ou la revendication 2 avec ledit composé à l'essai ; et
b) déterminer si ledit composé à l'essai provoque une hausse statistiquement significative de l'activité adénylcyclase intracellulaire ou de la concentration intracellulaire de calcium.

10. Procédé permettant de déterminer si un composé à l'essai est un ligand ou un agoniste de la protéine selon la revendication 1 ou la revendication 2, comprenant l'étape consistant à déterminer si une cellule exprimant une protéine comprenant la séquence d'acides aminés constituée fonctionnellement de SEQ ID NO . 1, internalise ladite protéine en réponse à un contact avec ledit composé à l'essai.

11. Procédé selon la revendication 10, selon lequel .
a) ladite protéine est exprimée par voie recombinante dans ladite cellule sous forme d'une protéine de fusion liée à une protéine fluorescente ;
b) la cellule de l'étape (a) est mise en contact avec ledit composé à l'essai ; et
c) l'internalisation de ladite protéine est déterminée par microscopie.

12. Procédé permettant de déterminer si un composé à l'essai est un antagoniste de la protéine selon la revendication 1 ou la revendication 2, comprenant les étapes consistant à :
a) incorporer une molécule d'ADN codant pour la protéine selon la revendication 1 ou la revendication 2 dans un vecteur d'expression de façon à ce qu'elle soit liée de manière opérationnelle à un promoteur ;
b) transfecter ledit vecteur d'expression dans un hôte ;
c) sélectionner les cellules transfectées dans l'étape b) qui ont une protéine constitutivement activée, comme en témoigne soit :
i) une hausse statistiquement significative de l'activité adénylcyclase intracellulaire ; soit
ii) une hausse statistiquement significative de la concentration intracellulaire de calcium ;
d) mettre les cellules sélectionnées dans l'étape c) en contact avec ledit composé à l'essai ; et
e) déterminer si ledit composé à l'essai provoque une baisse statistiquement significative de ladite activité adénylcyclase ou de ladite concentration de calcium par rapport à des cellules témoins n'ayant pas été mises en contact avec ledit composé à l'essai.

13. Procédé de dosage d'un composé à l'essai quant à son aptitude à altérer l'expression de la protéine selon la revendication 1 ou la revendication 2, comprenant les étapes consistant à :
a) cultiver des cellules exprimant la protéine selon la revendication 1 ou la revendication 2 ;
b) récolter lesdites cellules ; et
c) comparer l'expression protéique dans les cellules exposées audit composé à l'essai avec des cellules témoins cultivées dans des conditions essentiellement identiques mais n'ayant pas été exposées audit composé à l'essai.

14. Procédé selon la revendication 13, selon lequel lesdites cellules exprimant ladite protéine sont des cellules transformées par un vecteur d'expression comprenant une séquence polynucléotidique codant pour la protéine selon la revendication 1 ou la revendication 2.

15. Procédé selon la revendication 13 ou la revendication 14, selon lequel ledit composé à l'essai est un oligonucléotide d'au moins 15 nucléotides de long et qui comprend une séquence complémentaire à la séquence polynucléotidique codant pour ladite protéine.
